# EUROPEAN PATENT SPECIFICATION

(11) **EP 0 431 742 B1**
(45) Date of publication and mention of the grant of the patent: **16.08.1995**
(21) Application number: 90311732.3
(22) Date of filing: 25.10.1990
(51) Int. Cl.: A61L 9/12

(54) **Turbo-air freshener**
Turboluftverbesserungsvorrichtung
Appareil à turbine pour le rafraîchissement de l'air

(30) Priority: 04.12.1989 US 445273
(43) Date of publication of application: 12.06.1991
(73) Proprietor: STEINER COMPANY, INC., Chicago Illinois 60611 (US)
(72) Inventor: Holzner, Charles R., Chicago, Illinois 60657 (US); Voth, Allen J., Oak Park, Illinois 60302 (US)
(74) Representative: Newby, Martin John

(56) References cited:
- US-A- 3 990 848
- US-A- 4 035 451
- US-A- 4 059 422
- US-A- 4 743 406
- US-A- 4 840 770
- US-A- 4 931 224

## Description

### Background of the Invention

This invention relates in general to air freshening devices and, in particular, to a self-contained air freshener which draws ambient air through the apparatus, and about or through a deodorizing cartridge to vaporize materials contained in the cartridge for distribution into the air flow.

More specifically, but without restriction to the particular embodiment and/or use which is shown and described for purposes of illustration, this invention relates to a self-contained air freshener utilizing a replaceable cartridge containing or formed from a vaporizable material, and a replaceable battery power source having an improved air movement mechanism for increasing the flow of air through the vaporizable material. The replaceable or expendable items are carried within the apparatus to permit the convenient and selective replacement of these items when necessary.

Various types of air freshening or deodorizing devices have been utilized for inducing air flow past a product which may be vaporized, either by evaporation or sublimation, in order to distribute the vaporized product throughout the surrounding environment. To this end, apparatus such as disclosed in U.S. Patent Nos. 3,990,848 and 4,035,451 have been developed to distribute the vaporized product into the environment.

In U.S. Patent No. 3,990,848, an apparatus is disclosed which utilizes a self-contained disposable cartridge comprising a quantity of vaporizable product contained within a porous container upon which a battery power source is mounted and attached. In this manner, the entire unitary cartridge, product and power source, may be readily replaced with a new cartridge providing both a fresh power supply and a fresh quantity of deodorizer.

In the apparatus disclosed in U.S. Patent No. 4,035,451 a disposable cartridge, including both a quantity of material capable of being vaporized and a battery power source, is provided with the battery forming an integral part of the support structure for the cartridge. A strip material is folded in a convoluted configuration and concentrically spaced about the battery to define a series of parallel air passageways by which a product impregnated in the strip material is vaporized and distributed into the environment.

While each of these above-identified apparatus functions to distribute the vaporized product into the air, it has been found that the useful life of the vaporizable material and the useful life of the battery power source, are not necessarily the same. Therefore, when both the vaporizable material and the power source are integrated into the same unitary disposable and replaceable cartridge, the useful life of both is determined by the shortest useful life of either. This causes the operational life of the cartridge, as a whole, to be shorter than necessary.

In addition, these devices, as well as other such devices, are not conveniently serviceable after installation. Preferably such air fresheners are installed in isolated locations where a suitable air flow may be established, with the unit being installed above the unaided reach of a person to prevent vandalism. Such installations, however, have heretofore necessitated that the units be serviced while the service personnel are standing on a ladder or platform reaching into and working on the unit. Frequently such servicing is done by the service personnel working on the unit, above eye level, requiring servicing to be effected by feel.

The present invention is constructed such that all of the advantages of the device disclosed in U.S. Patent No. 4,743,406 issued May 10, 1988, the disclosure of which is herein specifically referred to, have been retained and in addition, improved air movement has been provided by as much as 30%.

### Summary of the Invention

It is therefore an object of this invention to provide improved air flow through self-contained air freshening or deodorizing devices.

Another object of this invention is to provide an anti-bootleg device for self-contained air freshening or deodorizing devices, the removal of which disables the device.

Still another object of this invention is to provide protection of the motor from air contaminants which may result in early failure of the motor.

A further object of this invention is to provide a self-contained air freshening device or deodorizing apparatus comprising a two-part housing including a mounting section for securing the apparatus in an operative position and a closure section for connection to the mounting section for forming an enclosure, battery-powered air movement generating means including a fan having a hub, a motor nested at least partially within the hub and connected thereto for rotating the fan hub generating a path of air flow through the enclosure, and air freshening or deodorizing means for supplying a source of vaporizable material to the path of air flow, the motor being protected from contaminants in the air by the hub substantially shielding said motor from the path of air flow.

Another object of the invention is to provide a self-contained air freshener or deodorizing apparatus comprising an enclosure vertically disposed in use having positioned therewithin a battery-powered air movement generating means including a fan having a hub, a motor nested at least partially within the hub and connected thereto for rotating the fan hub generating a path of air flow through the enclosure, means positioned below the motor for accelerating air in the path of air flow, and air freshening or deodorizing means for supplying a source of vaporizable material to the path of air flow.

Yet another object of the invention is to provide a self-contained air freshening or deodorizing apparatus comprising a two-part housing including a mounting section for securing the apparatus in an operative position and a plastic closure section for connection to the mounting section for forming an enclosure, battery-powered air movement generating means including a fan having a hub, a motor nested at least partially within the hub and connected thereto for rotating the fan hub generating a path of air flow through the enclosure in a direction away from the motor to protect the motor from contaminates in the air, a replaceable cartridge carrying an air freshening or deodorizing means for supplying a source of vaporizable material to the path of air flow, interference means including a mounting plate having an aperture therein for mounting the interference means to the plastic closure section and a prong extending toward and into the replaceable cartridge, a portion of the plastic closure section extending through the aperture in the mounting plate fixedly to mount the interference means to the closure section, and receptacle means on the cartridge for receiving the interference prong to permit the two part housing to be closed, whereby cartridges without the receptacle means being prevented from fitting within the closure section and preventing formation of an enclosure.

The invention consists of certain novel features and a combination of parts hereinafter fully described, illustrated in the accompanying drawings, and particularly pointed out in the appended claims.

### Brief Description of the Drawings

For the purpose of facilitating an understanding of the invention, there is illustrated in the accompanying drawings a preferred embodiment thereof, from an inspection of which, when considered in connection with the following description, the invention, its construction and operation, and many of its advantages should be readily understood and appreciated.
FIG. 1 is a cross-sectional view of the self-contained deodorizing device of the present invention without the deodorizing cartridge and the battery;
FIG. 2 is a top view of the self-contained air freshening device of FIG. 1 with the top removed;
FIG. 3 is a view like FIG. 1 with the cartridge and battery in place;
FIG. 4 is an enlarged view of a strut with air accelerating mechanism;
FIG. 5 is an enlarged view of the shroud and air movement device of the present invention;
FIG. 6 is a perspective view of the shroud and motor housing;
FIG. 7 is a bottom elevational view of the shroud illustrated in FIG. 6 with the motor present;
FIG. 8 is an elevational view of the propeller portion of the air movement device;
FIG. 9 is an elevational view of a deodorizing cartridge; and
FIG. 10 is a sectional view of the cartridge illustrated in FIG. 9 as seen along lines 10-10 thereof.

### Description of the Preferred Embodiment

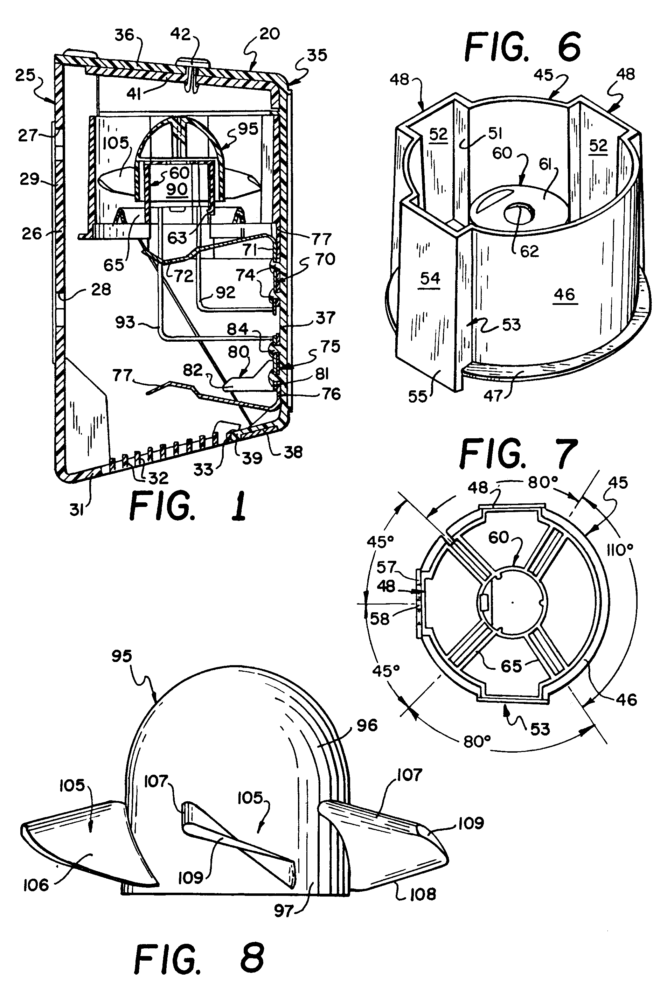

Referring now to FIGS. 1-3, there is illustrated a self-contained air freshening or deodorizing device 20 which is formed in a substantially rectangular shape. The deodorizing device 20 comprises a two-part housing including a mounting section 25 adapted to secure the device to a vertical wall, and a closure section 35 which, when connected to the mounting section 25, together form a rectangular shaped enclosure. The mounting section 25 includes a rectangular plate or wall portion 26 having a pair of spaced upper openings or apertures 27 and a lower aperture 28 formed therein by which the mounting section 25 may be secured to a vertical surface, or the mounting section can be attached to a wall by means of a pressure-adhesive-backed foam tape 29. The mounting section 25 further includes an upwardly extending bottom 31 having a plurality of vents 32 centrally positioned therein and having a locking aperture 33 just outside of the vents 32.

The closure section 35 fits onto and locks with the mounting section 25 in the same way as shown in the US-'406 patent and will not be redescribed here. The closure section 35 has a top 36 and a vertically positioned front wall 37 having a short inwardly extending bottom wall 38 terminating in a tang 39 adapted to snap fit within the locking aperture 33 so as to form the enclosure required for operation of the deodorizing or air freshening device 20. The closure section 35 is completed by a baffle plate 41 and control member 42 therefor positioned in the top wall 36.

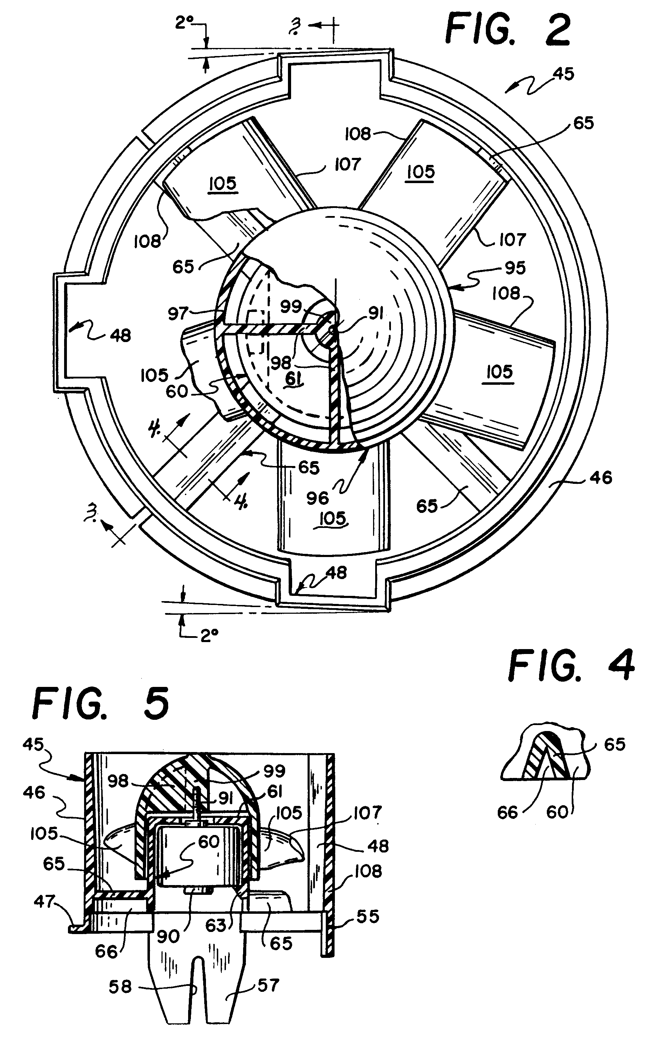

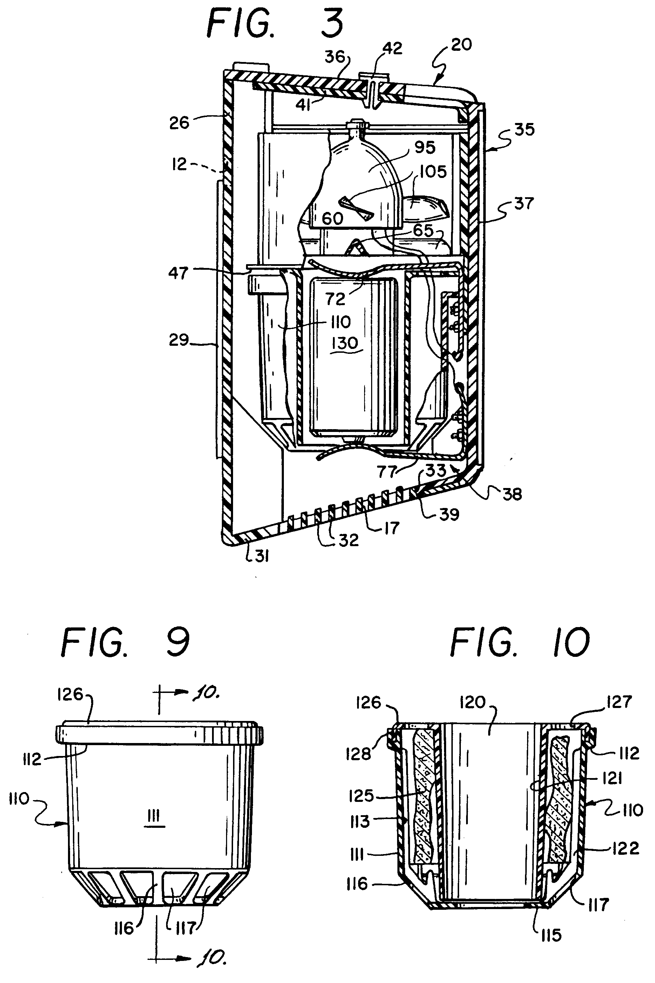

A shroud 45 is generally cylindrical in shape and has a cylindrical wall 46 terminating in an outwardly extending flange 47. Two chutes 48 in the form of rectangularly extending cut-outs each having opposed side walls 51 and interconnected by a bight or recess wall 52 are positioned circumferentially 90° from each other. The shroud 45 also has a chute 53 having the same general construction as the chutes 48 and having a recessed or bight wall 54, the difference being that the chute 53 has a downwardly extending portion 55 of the recessed wall 54. Mounting apertures (56) are in the chute 48 as best seen in Fig. 7 and a mounting flange 57 having a slot 58 extends downwardly from one of the chutes 48 as best seen in FIG. 5. The mounting flange 57 with slot 58 along with the apertures 56 are used to mount the shroud 45 to the inside of the front wall 37 of the closure sections 35.

Positioned within the shroud 45 is a cylindrical motor housing 60 having a top 61 circular in plan view having a central aperture 62 therein, for a purpose hereinafter set forth, and a downwardly extending retaining clip 63. The cylindrical motor housing 60 is maintained centrally disposed within the shroud 45 by means of a series of struts 65. Each of the struts 65 as best seen in FIGS. 2, 4 and 5 is in the shape of an inverted V and provides a raceway 66 which is downwardly facing in use, for electrical connections between the motor and battery. There are four struts 65 each interconnecting the cylindrical motor housing 60 with the interior surface of the shroud 45 and each being inverted V-shaped in transverse cross section. As seen, the two right-hand struts are spaced 110° apart, center to center, and the left-hand struts are spaced 90° apart.

Mounted to the front wall 37 of the closure section 35 is an upper battery clip 70. The upper battery clip 70 has a mounting portion 71 which is relatively straight and is provided with a pair of apertures (not numbered) and an inwardly extending angularly disposed battery engaging portion 72. The battery clip 70 is mounted to the front wall 37 by means of a pair of plastic bosses 74 which extend through the apertures in the battery clip and are deformed so as to form the bosses as shown. Similarly, a lower battery clip 75 is mounted to the front wall 37 in a position vertically spaced from the upper battery clip 70. The lower battery clip 75 also has a mounting portion 76 with a pair of vertically spaced apart apertures, not numbered, and a battery engaging portion 77. Mounted in an abutting relationship with the lower battery clip 75 is an anti-bootleg interference device 80. The anti-bootleg interference device is shaped like that disclosed in the copending U.S. Patent No. 4,931,224, issued June 5, 1990 entitled "Air Freshener". The anti-bootleg interference device 80 which may be the same as that disclosed in the copending application has a vertically extending mounting plate 81 and a pair of inwardly extending prongs 82. Both the anti-bootleg device 80 and the lower battery clip 75 are fixedly mounted to the front wall 37 by the portion of plastic which extends through the apertures in each of the clip 75 and the anti-bootleg device 80 and are deformed into the bosses 84 as shown. Mounting the anti-bootleg device 80 in this manner precludes removal of the anti-bootleg device without destruction of the bosses 74 or 84 and hence, destruction of the battery clip 70 or 75, respectively, resulting in an inoperable device. This is an important feature of this invention.

A motor 90 is positioned within the motor housing 60 and retained in place by the retaining clip 63. The motor 90 has an output shaft 91 which extends through the central aperture 62 in the cylindrical motor housing 60 and has a pair of leads 92 and 93 electrically connected to the upper battery clip 70 and the lower battery clip 75. Mounted on the output shaft 91 of the motor 90 is a propeller or air movement device 95. The propeller 95, as best seen in FIGS. 1, 2, 5 and 8 has a hub 96, the lower portion of which is cylindrical and the inside of which is provided with four ribs 98 meeting in a centrally positioned sleeve 99 adapted to receive therein the output shaft 91 from the motor 90. The fit between the sleeve 99 and the output shaft 91 is sufficiently tight to ensure that the propeller 95 rotates upon operation of the motor 90. The propeller 95 has five blades 105 positioned circumferentially around the cylindrical section 97 of hub 96. Each of the blades 105 has a base portion 106, a leading edge 107 and a trailing edge 108. The leading edge 107 of each of the blades 105 is thicker than the trailing edge 108 and provides an aerodynamic surface to ensure efficient air movement upon rotation of the propeller 95. The pitch of each of the blades 105 may be selected depending upon the amount of air to be moved but as illustrated, there is approximately a 20° angle between the inside edge formed between the juncture of the base portion 106 and the hub 95 and the outside edge 109 of each blade 105. It has been found that the design of the blades 105 as illustrated in combination with using five such blades results in moving approximately 30% more air than heretofore possible with the design of the US-'406 patent.

The cartridge 110 which is fundamentally the same as that disclosed in the US-'406 and US-'224 patents, includes a cylindrical body 111 having a lip 112 at the upper end thereof and an inner surface 113. A base ring 115 is joined to the cylindrical body 111 by a tapered wall 116 provided with a series of openings 117 therein. A well 120 is positioned inside the cylindrical body 111 and extends downwardly and terminates at a point slightly above the base ring 115, the well 120 having an inner wall 121 which is generally cylindrical. The well 120 forms an annular space with the inner surface 113 of the cylindrical body 111 to receive a vaporizable material 125 usually in a packet having a semipermeable covering, the vaporizable material 125 being maintained in the annular space by a plurality of ribs 122 which extend inwardly from the cylindrical wall or body 111. The well 120 has a top 126 which has as plurality of openings 127 therein and a downwardly extending lip 128 which fits within the upwardly extending lip 112 of the cylindrical body 111. The lips 112 and 128 are dimensioned for friction welding so as to complete the cartridge 110. A battery 130 ((see FIG. 3) loosely fits within the well 120 and in use is positioned in electrical contact with the upper and lower battery clips 70 and 75 to provide power to the electrical motor 90.

In operation, a cartridge 110 containing deodorizing material 125 has a battery 130 inserted in the well or battery chamber 120, and the cartridge and battery are inserted into the closure section 35 and particularly between the upper and lower battery clips 70 and 75. Because the battery clips 70 and 75 are connected by means of wires 92 and 93 to the motor 90, an electric circuit is established between the motor 90 and the battery 130 to power the propeller 95. The fan motor 90 will run until such time as it is necessary to replace the battery power source. However, when replacing the discharged battery 130, if it is found that deodorizing material 125 is still contained within the cartridge 110, the battery 130 may be replaced merely by removing the cartridge 110 and inserting a fresh battery in place of the one that has been discharged. In this manner, the entire cartridge 110 does not need to be replaced merely because the battery 130 has been discharged. Similarly, if it is found that the vaporizable material 125 has been expended, the battery 130 may merely be removed from the cartridge 10 containing the expended material 125 and the battery inserted into a new cartridge 110 containing fresh vaporizable material 125.

An important aspect of the present invention is the provision of the propeller 95 having five pitched blades 105 each having an aerodynamically efficient surface with an enlarged leading edge 107 and a thinner trailing edge 108 with the pitch being as disclosed so as to provide in combination with the struts 65 each of which accelerate air as it flows thereby 30 percent greater air movement in the device of the invention as compared to the device of the '406 patent. Another important aspect of the invention is the placement of the motor 90 within the hub 96 to conserve space and allow for larger fan blades 105 to move more air. An unexpected feature of placing the motor 90 within the hub 96 is that the motor is protected from contaminants in the air, which is particularly important in beauty shops where there may be large quantities of hair spray and other materials which can foul a motor.

## Claims

1. A self-contained air freshening or deodorizing apparatus (20) including a housing (25, 35) forming an enclosure, battery-powered air movement generating means (90, 95, 96) within said enclosure including a fan (95) for generating air flow through said enclosure, and air freshening or deodorizing means (110) for supplying a source of vaporizable material (125) in the path of said air flow, characterized by said fan having a hub and said motor (90) being nested at least partially within said hub and connected thereto for rotating said fan, said motor being protected from contaminants in the air by said hub substantially shielding said motor from the path of air flow.

2. The self-contained air freshener or deodorizing apparatus of claim 1, wherein said hub has a dome shaped outer surface and forms an interior compartment for receiving said motor.

3. The self-contained air freshener or deodorizing apparatus of claim 2, wherein said motor has an output shaft (91) frictionally engaging the inside of said hub to rotate same.

4. The self-contained air freshener or deodorizing apparatus of claim 2, wherein said hub outer surface is part cylindrical and has five fan blades (105) extending outwardly from said cylindrical surface, each of said fan blades having a base (106) angularly disposed to the vertical and a free end angularly rotated with respect to said blade base.

5. The self-contained air freshener or deodorizing apparatus of claim 4, wherein each blade has a leading edge (107) thicker than the trailing edge (108), and wherein the bottom surface of each blade is at least partially arcuate.

6. The self-contained air freshener or deodorizing apparatus of claim 1, and further comprising a motor housing (60) positioned inside said hub having a substantially closed top and retaining means (63) for retaining said motor fixedly in place within said motor housing during operation of said fan.

7. The self-contained air freshener or deodorizing apparatus of claim 1, including a shroud (45) surrounding said fan having a plurality of radially extending struts (65) positioned in use below said fan, each of said struts having a surface facing said fan which accelerates air passing across said strut surface.

8. The self-contained air-freshener or deodorizing apparatus of claim 7, wherein at least some of said struts (65) are inverted V-shaped in transverse cross-section to provide raceways (66) for electrical connections between said motor and a battery disposed in said enclosure.

9. The self-contained air freshener or deodorizing apparatus of claim 7 wherein said shroud is cylindrical with at least one (48) vertically extending abutment in the inner surface thereof.

10. The self-contained air freshener or deodorizing apparatus of claim 1, characterized by a replaceable cartridge carrying an air freshening or deodorizing material for supplying said source of vaporizable material to said path of air flow, and interference means including a mounting plate (81) mounted on said closure section and having at least one prong fixedly positioned within said housing to intrude into space occupied by said cartridge, and receptacle means on said cartridge for receiving said interference prong to permit insertion of a cartridge into said housing whereby cartridges without said receptacle means are prevented from fitting within said housing by said interference means.

## Patentansprüche

1. In sich abgeschlossene Luftauffrisch- oder Desodorierungsvorrichtung (20), die folgendes enthält: ein eine Einfassung bildendes Gehäuse (25, 35), ein batteriegespeistes Luftbewegungserzeugungsmittel (90, 95, 96) in der Einfassung, das ein Gebläse (95) zur Erzeugung eines Luftstroms durch die Einfassung enthält, und ein Luftauffrisch- oder Desodorierungsmittel (110) zur Zufuhr einer Quelle verdunstungsfähigen Materials (125) in den Weg des Luftstroms, dadurch gekennzeichnet, daß das Gebläse eine Nabe aufweist und der Motor (90) zumindest teilweise in der Nabe untergebracht und damit verbunden ist, um das Gebläse zu drehen, wobei der Motor von in der Luft enthaltenen Verunreinigungen geschützt wird, indem die Nabe den Motor im wesentlichen von dem Luftstromweg abschirmt.

2. In sich abgeschlossene Luftauffrisch- oder Desodorierungsvorrichtung nach Anspruch 1, bei der die Nabe eine kalottenförmige Außenfläche aufweist und ein Innenfach zur Aufnahme des Motors bildet.

3. In sich abgeschlossene Luftauffrisch- oder Desodorierungsvorrichtung nach Anspruch 2, bei der die Abtriebswelle (91) des Motors mit der Innenseite der Nabe in Reibungseingriff steht, um sie anzutreiben.

4. In sich abgeschlossene Luftauffrisch- oder Desodorierungsvorrichtung nach Anspruch 2, bei der die Außenfläche der Nabe teilweise zylindrisch ist und fünf Gebläseflügel (105) aufweist, die sich von der zylindrischen Fläche nach außen erstrecken, wobei jeder der Gebläseflügel eine Basis (106), die in einem Winkel zur Vertikalen steht, und ein freies Ende aufweist, das gegenüber der Flügelbasis in einem Winkel gedreht ist.

5. In sich abgeschlossene Luftauffrisch- oder Desodorierungsvorrichtung nach Anspruch 4, bei der jeder Flügel eine Vorderkante (107) aufweist, die dicker als die Hinterkante (108) ist, und bei der die Unterfläche jedes Flügels zumindest teilweise bogenförmig ist.

6. In sich abgeschlossene Luftauffrisch- oder Desodorierungsvorrichtung nach Anspruch 1, die weiterhin ein in der Nabe angebrachtes Motorgehäuse (60) umfaßt, das einen im wesentlichen geschlossenen Oberteil und ein Haltemittel (63) zum Festhalten des Motors im Motorgehäuse bei Betrieb des Gebläses.

7. In sich abgeschlossene Luftauffrisch- oder Desodorierungsvorrichtung nach Anspruch 1, die eine das Gebläse umgebende Verkleidung (45) mit mehreren sich radial erstreckenden Streben (65) enthält, die sich bei Gebrauch unterhalb des Gebläses befinden, wobei jede der Streben eine zum Gebläse weisende Fläche aufweist, wodurch die über die Strebenfläche strömende Luft beschleunigt wird.

8. In sich abgeschlossene Luftauffrisch- oder Desodorierungsvorrichtung nach Anspruch 7, bei der zumindest einige der Streben (65) im Querschnitt dachförmig sind, um Kanäle (66) für elektrische Verbindungen zwischen dem Motor und einer Batterie, die in der Einfassung angeordnet sind, zu bilden.

9. In sich abgeschlossene Luftauffrisch- oder Desodorierungsvorrichtung nach Anspruch 7, bei der die Verkleidung zylindrisch ist und in ihrer Innenfläche mindestens eine (48) sich vertikal erstreckende Stoßfläche aufweist.

10. In sich abgeschlossene Luftauffrisch- oder Desodorierungsvorrichtung nach Anspruch 1, gekennzeichnet durch eine auswechselbare Patrone, die ein Luftauffrisch- oder Desodorierungsmaterial enthält, um die Quelle verdunstungsfähigen Materials dem Luftstromweg zuzuführen, und durch ein Sperrmittel, das eine an dem Schließabschnitt angebrachte Befestigungsplatte (81) enthält, die mindestens eine in dem Gehäuse fest positionierte Klaue aufweist, die in den von der Patrone eingenommenen Raum hineinragt, und durch Aufnahmemittel an der Patrone zur Aufnahme der Sperrklaue, um ein Einsetzen einer Patrone in das Gehäuse zu gestatten, wodurch Patronen ohne das Aufnahmemittel aufgrund des Sperrmittels nicht in das Gehäuse passen.

## Revendications

1. Appareil (20) autonome de rafraîchissement ou de désodorisation de l'air incluant un logement (25, 35) formant une enceinte, un moyen (90, 95, 96) à l'intérieur de ladite enceinte alimenté par pile pour engendrer un déplacement d'air, comprenant un ventilateur (95) pour engendrer un courant d'air à travers ladite enceinte, et un moyen (110) de rafraîchissement ou de désodorisation de l'air pour alimenter la voie dudit courant d'air à partir d'une source de matériau vaporisable (125), caractérisé en ce que ledit ventilateur comporte un moyeu et ledit moteur (90) étant logé au moins partiellement à l'intérieur dudit moyeu et y étant relié pour faire tourner ledit ventilateur, ledit moteur étant protégé contre les agents viciateurs de l'air par le fait que ledit moyeu abrite substantiellement ledit moteur de la voie du courant d'air.

2. L'appareil autonome de rafraîchissement ou de désodorisation de l'air de la revendication 1, caractérisé en ce que ledit moyeu comporte une surface externe en forme de dôme et forme un logement intérieur pour recevoir ledit moteur.

3. L'appareil autonome de rafraîchissement ou de désodorisation de l'air de la revendication 2, caractérisé en ce que ledit moteur comporte un arbre de sortie (91) en contact par friction avec l'intérieur dudit moyeu pour faire tourner ce dernier.

4. L'appareil autonome de rafraîchissement ou de désodorisation de l'air de la revendication 2, caractérisé en ce que ladite surface externe du moyeu est en partie cylindrique et comporte cinq ailettes (105) de ventilateur s'étendant vers l'extérieur depuis ladite surface cylindrique, chacune desdites ailettes de ventilateur comportant une embase (106) disposée diagonalement par rapport à la verticale et une extrémité libre fait tourner diagonalement par rapport à ladite embase d'ailette.

5. L'appareil autonome de rafraîchissement et de désodorisation de l'air de la revendication 4, caractérisé en ce que chaque ailette comporte un bord d'attaque (107) plus épais que le bord de fuite (108), et en ce que la surface inférieure de chaque ailette est au moins partiellement en forme d'arc.

6. L'appareil autonome de rafraîchissement ou de désodorisation de l'air de la revendication 1, et comprenant de plus un logement de moteur (60) positionné à l'intérieur dudit moyeu comportant une partie supérieure substantiellement fermée et un moyen de retenue (63) pour retenir le moteur solidement en place à l'intérieur dudit logement de moteur durant le fonctionnement dudit ventilateur.

7. L'appareil autonome de rafraîchissement ou de désodorisation de l'air de la revendication 1, incluant une enveloppe (45) entourant ledit ventilateur comportant une pluralité de traverses (65) s'étendant radialement et positionnées en fonctionnement sous ledit ventilateur, chacune desdites traverses comportant une surface en vis-à-vis dudit ventilateur qui accélère l'air passant en travers de ladite surface de traverse.

8. L'appareil autonome de rafraîchissement ou de désodorisation de l'air de la revendication 7, caractérisé en ce qu'au moins quelques-unes desdites traverses (65) sont en forme de V renversé en coupe transversale pour fournir des passages (66) pour les raccordements électriques entre ledit moteur et une pile située dans ladite enceinte.

9. L'appareil autonome de rafraîchissement ou de désodorisation de l'air de la revendication 7, caractérisé en ce que ladite enveloppe est cylindrique avec au moins un (48) aboutement s'étendant verticalement dans la surface interne de cette dernière.

10. L'appareil autonome de rafraîchissement ou de déodorisation de l'air de la revendication 1, caractérisé par une cartouche remplaçable contenant un matériau de rafraîchissement ou de désodorisation de l'air pour alimenter ladite voie du courant d'air à partir de ladite source de matériau vaporisable, et par un moyen d'intrusion incluant une plaque de fixation (81) montée sur ladite section de fermeture et comportant au moins un fourchon positionné de façon fixe à l'intérieur dudit logement pour faire saillie dans l'espace occupé par ladite cartouche, et par un moyen de réceptacle sur ladite cartouche pour recevoir ledit fourchon d'intrusion pour permettre l'insertion d'une cartouche dans ledit logement, les cartouches sans dit moyen de réceptacle ayant ainsi leur insertion interdite à l'intérieur dudit logement par ledit moyen d'intrusion.
